# EUROPEAN PATENT APPLICATION

(11) **EP 1 437 363 A1**
(43) Date of publication of application: **14.07.2004**
(21) Application number: 02800251.7
(22) Date of filing: 26.09.2002
(51) Int. Cl.: C07K 14/47, C12N 15/12, C12N 1/21, C12N 1/19, C12N 5/10, C07K 16/18

(54) **DRUG TRANSPORTER AND USE THEREOF**

(30) Priority: 28.09.2001 JP 2001302830
(71) Applicant: Proteinexpress Co., Ltd., Chosi-shi, Chiba 288-0041 (JP); Kazusa Dna Research Institute, Kisarazu-shi, Chiba 292-0818 (JP); Fujimura, Akio, Kawachi-gun, Tochigi 329-0434 (JP); Tsuruoka, Shuichi, Kawachi-gun, Tochigi 329-0434 (JP); Ishibashi, Kenichi, Tokyo 167-0031 (JP); Imai, Masashi, Oyama-shi, Tochigi 323-0807 (JP)
(72) Inventor: FUJIMURA, Akio, Kawachi-gun, Tochigi 329-0434 (JP); TSURUOKA, Shuichi, Kawachi-gun, Tochigi 329-0434 (JP); ISHIBASHI, Kenichi, Suginami-ku, Tokyo 167-0031 (JP); IMAI, Masashi, Oyama-shi, Tochigi 323-0807 (JP); OHARA, Osamu, c/o Kazusa DNA Research Institute, Kisarazu-shi, Chiba 292-0818 (JP); NAGASE, Takahiro, c/o Kazusa DNA Research Inst., Kisarazu-shi, Chiba 292-0818 (JP)
(74) Representative: Curtis, Philip Anthony
(86) International application number: PCT/JP2002/009960
(87) International publication number: WO 2003/029290

(57) **Abstract**

It is an object of the present invention to provide a protein having a drug transport activity, a method for screening a compound that promotes or inhibits the activity of the drug transporter, a compound obtained by the method, an antibody against the drug transporter, a pharmaceutical composition comprising the same, or the like. The protein with a drug transport activity of the present invention has an amino acid sequence represented by SEQ ID NO: 1.

SEQUENCE LISTING

<110> Protein Express Co., Ltd.
      Kazusa DNA Research Institute
      FUJIMURA, Akio
      TSURUOKA, Shuichi
      ISHIBASHI, Kenichi
      IMAI, Masashi

<120> Drug transpoter, and its use

<130> pctf140

<140>
<141>

<150> JP 2001-302830
<151> 2001-09-28

<160> 2

<170> PatentIn Ver. 2.1

<210> 1
<211> 1581

<212> PRT

<213> Homo sapiens

<400> 1

Met Arg Lys Arg Lys Ile Ser Val Cys Gln Gln Thr Trp Ala Leu Leu
1               5                   10                  15

Cys Lys Asn Phe Leu Lys Lys Trp Arg Met Lys Arg Glu Ser Leu Met
            20                  25                  30

Glu Trp Leu Asn Ser Leu Leu Leu Leu Leu Cys Leu Tyr Ile Tyr Pro
        35                  40                  45

His Ser His Gln Val Asn Asp Phe Ser Ser Leu Leu Thr Met Asp Leu
    50                  55                  60

Gly Arg Val Asp Thr Phe Asn Glu Ser Arg Phe Ser Val Val Tyr Thr
65                  70                  75                  80

Pro Val Thr Asn Thr Thr Gln Gln Ile Met Asn Lys Val Ala Ser Thr
                85                  90                  95

Pro Phe Leu Ala Gly Lys Glu Val Leu Gly Leu Pro Asp Glu Glu Ser
            100                 105                 110

Ile Lys Glu Phe Thr Ala Asn Tyr Pro Glu Glu Ile Val Arg Val Thr

        115                 120                 125

Phe Thr Asn Thr Tyr Ser Tyr His Leu Lys Phe Leu Leu Gly His Gly

    130                 135                 140

Met Pro Ala Lys Lys Glu His Lys Asp His Thr Ala His Cys Tyr Glu

145                 150                 155                 160

Thr Asn Glu Asp Val Tyr Cys Glu Val Ser Val Phe Trp Lys Glu Gly

                165                 170                 175

Phe Val Ala Leu Gln Ala Ala Ile Asn Ala Ala Ile Ile Glu Ile Thr

            180                 185                 190

Thr Asn His Ser Val Met Glu Glu Leu Met Ser Val Thr Gly Lys Asn

        195                 200                 205

Met Lys Met His Ser Phe Ile Gly Gln Ser Gly Val Ile Thr Asp Leu

    210                 215                 220

Tyr Leu Phe Ser Cys Ile Ile Ser Phe Ser Ser Phe Ile Tyr Tyr Ala

225                 230                 235                 240

Ser Val Asn Val Thr Arg Glu Arg Lys Arg Met Lys Ala Leu Met Thr

                245                 250                 255

Met Met Gly Leu Arg Asp Ser Ala Phe Trp Leu Ser Trp Gly Leu Leu
            260                 265                 270
Tyr Ala Gly Phe Ile Phe Ile Met Ala Leu Phe Leu Ala Leu Val Ile
        275                 280                 285
Arg Ser Thr Gln Phe Ile Ile Leu Ser Gly Phe Met Val Val Phe Ser
    290                 295                 300
Leu Phe Leu Leu Tyr Gly Leu Ser Leu Val Ala Leu Ala Phe Leu Met
305                 310                 315                 320
Ser Ile Leu Val Lys Lys Ser Phe Leu Thr Gly Leu Val Val Phe Leu
                325                 330                 335
Leu Thr Val Phe Trp Gly Cys Leu Gly Phe Thr Ser Leu Tyr Arg His
            340                 345                 350
Leu Pro Ala Ser Leu Glu Trp Ile Leu Ser Leu Leu Ser Pro Phe Ala
        355                 360                 365
Phe Met Leu Gly Met Ala Gln Leu Leu His Leu Asp Tyr Asp Leu Asn
    370                 375                 380
Ser Asn Ala Phe Pro His Pro Ser Asp Gly Ser Asn Leu Ile Val Ala
385                 390                 395                 400

Thr Asn Phe Met Leu Ala Phe Asp Thr Cys Leu Tyr Leu Ala Leu Ala
                405                 410                 415
Ile Tyr Phe Glu Lys Ile Leu Pro Asn Glu Tyr Gly His Arg Arg Pro
            420                 425                 430
Pro Leu Phe Phe Leu Lys Ser Ser Phe Trp Ser Gln Thr Gln Lys Thr
        435                 440                 445
Asp His Val Ala Leu Glu Asp Glu Met Asp Ala Asp Pro Ser Phe His
    450                 455                 460
Asp Ser Phe Glu Gln Ala Pro Pro Glu Phe Gln Gly Lys Glu Ala Ile
465                 470                 475                 480
Arg Ile Arg Asn Val Thr Lys Glu Tyr Lys Gly Lys Pro Asp Lys Ile
                485                 490                 495
Glu Ala Leu Lys Asp Leu Val Phe Asp Ile Tyr Glu Gly Gln Ile Thr
            500                 505                 510
Ala Ile Leu Gly His Ser Gly Ala Gly Lys Ser Thr Leu Leu Asn Ile
        515                 520                 525
Leu Ser Gly Leu Ser Val Pro Thr Lys Gly Ser Val Thr Ile Tyr Asn

    530                 535                 540

Asn Lys Leu Ser Glu Met Ala Asp Leu Glu Asn Leu Ser Lys Leu Thr
545                 550                 555                 560

Gly Val Cys Pro Gln Ser Asn Val Gln Phe Asp Phe Leu Thr Val Arg
                565                 570                 575

Glu Asn Leu Arg Leu Phe Ala Lys Ile Lys Gly Ile Leu Pro Gln Glu
            580                 585                 590

Val Asp Lys Glu Ile Phe Leu Leu Asp Glu Pro Thr Ala Gly Leu Asp
        595                 600                 605

Pro Phe Ser Arg His Gln Val Trp Asn Leu Leu Lys Glu Arg Lys Thr
    610                 615                 620

Asp Arg Val Ile Leu Phe Ser Thr Gln Phe Met Asp Glu Ala Asp Ile
625                 630                 635                 640

Leu Ala Asp Arg Lys Val Phe Leu Ser Gln Gly Lys Leu Lys Cys Ala
                645                 650                 655

Gly Ser Ser Leu Phe Leu Lys Lys Lys Trp Gly Ile Gly Tyr His Leu
            660                 665                 670

Ser Leu Gln Leu Asn Glu Ile Cys Val Glu Glu Asn Ile Thr Ser Leu
        675                 680                 685
Val Lys Gln His Ile Pro Asp Ala Lys Leu Ser Ala Lys Ser Glu Gly
    690                 695                 700
Lys Leu Ile Tyr Thr Leu Pro Leu Glu Arg Thr Asn Lys Phe Pro Glu
705                 710                 715                 720
Leu Tyr Lys Asp Leu Asp Ser Tyr Pro Asp Leu Gly Ile Glu Asn Tyr
                725                 730                 735
Gly Val Ser Met Thr Thr Leu Asn Glu Val Phe Leu Lys Leu Glu Gly
            740                 745                 750
Lys Ser Thr Ile Asn Glu Ser Asp Ile Ala Ile Leu Gly Glu Val Gln
        755                 760                 765
Ala Glu Lys Ala Asp Asp Thr Glu Arg Leu Val Glu Met Glu Gln Val
    770                 775                 780
Leu Ser Ser Leu Asn Lys Met Arg Lys Thr Ile Gly Gly Val Ala Leu
785                 790                 795                 800
Trp Arg Gln Gln Ile Cys Ala Ile Ala Arg Val Arg Leu Leu Lys Leu
                805                 810                 815

Lys His Glu Arg Lys Ala Leu Leu Ala Leu Leu Leu Ile Leu Met Ala
            820                 825                 830
Gly Phe Cys Pro Leu Leu Val Glu Tyr Thr Met Val Lys Ile Tyr Gln
        835                 840                 845
Asn Ser Tyr Thr Trp Glu Leu Ser Pro His Leu Tyr Phe Leu Ala Pro
    850                 855                 860
Gly Gln Gln Pro His Asp Pro Leu Thr Gln Leu Leu Ile Ile Asn Lys
865                 870                 875                 880
Thr Gly Ala Ser Ile Asp Asp Phe Ile Gln Ser Val Glu His Gln Asn
                885                 890                 895
Ile Ala Leu Glu Val Asp Ala Phe Gly Thr Arg Asn Gly Thr Asp Asp
            900                 905                 910
Pro Ser Tyr Asn Gly Ala Ile Thr Val Cys Cys Asn Glu Lys Asn Tyr
        915                 920                 925
Ser Phe Ser Leu Ala Cys Asn Ala Lys Arg Leu Asn Cys Phe Pro Val
    930                 935                 940
Leu Met Asp Ile Val Ser Asn Gly Leu Leu Gly Met Val Lys Pro Ser

945                 950                 955                 960

Val His Ile Arg Thr Glu Arg Ser Thr Phe Leu Glu Asn Gly Gln Asp

                965                 970                 975

Asn Pro Ile Gly Phe Leu Ala Tyr Ile Met Phe Trp Leu Val Leu Thr

            980                 985                 990

Ser Ser Cys Pro Pro Tyr Ile Ala Met Ser Ser Ile Asp Asp Tyr Lys

        995                 1000                1005

Asn Arg Ala Arg Ser Gln Leu Arg Ile Ser Gly Leu Ser Pro Ser Ala

    1010                1015                1020

Tyr Trp Phe Gly Gln Ala Leu Val Asp Val Ser Leu Tyr Phe Leu Val

1025                1030                1035                1040

Phe Val Phe Ile Tyr Leu Met Ser Tyr Ile Ser Asn Phe Glu Asp Met

                1045                1050                1055

Leu Leu Thr Ile Ile His Ile Ile Gln Ile Pro Cys Ala Val Gly Tyr

            1060                1065                1070

Ser Phe Ser Leu Ile Phe Met Thr Tyr Val Ile Ser Phe Ile Phe Arg

        1075                1080                1085

Lys Gly Arg Lys Asn Ser Gly Ile Trp Ser Phe Cys Phe Tyr Val Val
    1090                1095                1100

Thr Val Phe Ser Val Ala Gly Phe Ala Phe Ser Ile Phe Glu Ser Asp
1105                1110                1115                1120

Ile Pro Phe Ile Phe Thr Phe Leu Ile Pro Pro Ala Thr Met Ile Gly
                1125                1130                1135

Cys Leu Phe Leu Ser Ser His Leu Leu Phe Ser Ser Leu Phe Ser Glu
            1140                1145                1150

Glu Arg Met Asp Val Gln Pro Phe Leu Val Phe Leu Ile Pro Phe Leu
        1155                1160                1165

His Phe Ile Ile Phe Leu Phe Thr Leu Arg Cys Leu Glu Trp Lys Phe
    1170                1175                1180

Gly Lys Lys Ser Met Arg Lys Asp Pro Phe Phe Arg Ile Ser Pro Arg
1185                1190                1195                1200

Ser Ser Asp Val Cys Gln Asn Pro Glu Glu Pro Glu Gly Glu Asp Glu
                1205                1210                1215

Asp Val Gln Met Glu Arg Val Arg Thr Ala Asn Ala Leu Asn Ser Thr
            1220                1225                1230

Asn Phe Asp Glu Lys Pro Val Ile Ile Ala Ser Cys Leu Arg Lys Glu
        1235                1240                1245
Tyr Ala Gly Lys Arg Lys Gly Cys Phe Ser Lys Arg Lys Asn Lys Ile
    1250                1255                1260
Ala Thr Arg Asn Val Ser Phe Cys Val Arg Lys Gly Glu Val Leu Gly
1265                1270                1275                1280
Leu Leu Gly His Asn Gly Ala Gly Lys Ser Thr Ser Ile Lys Val Ile
                1285                1290                1295
Thr Gly Asp Thr Lys Pro Thr Ala Gly Gln Val Leu Leu Lys Gly Ser
            1300                1305                1310
Gly Gly Gly Asp Ala Leu Glu Phe Leu Gly Tyr Cys Pro Gln Glu Asn
        1315                1320                1325
Ala Leu Trp Pro Asn Leu Thr Val Arg Gln His Leu Glu Val Tyr Ala
    1330                1335                1340
Ala Val Lys Gly Leu Arg Lys Gly Asp Ala Glu Val Ala Ile Thr Arg
1345                1350                1355                1360
Leu Val Asp Ala Leu Lys Leu Gln Asp Gln Leu Lys Ser Pro Val Lys

                1365                1370                1375

Thr Leu Ser Glu Gly Ile Lys Arg Lys Leu Cys Phe Val Leu Ser Ile

            1380                1385                1390

Leu Gly Asn Pro Ser Val Val Leu Leu Asp Glu Pro Ser Thr Gly Met

        1395                1400                1405

Asp Pro Glu Gly Gln Gln Gln Met Trp Gln Ala Ile Arg Ala Thr Phe

    1410                1415                1420

Arg Asn Thr Glu Arg Gly Ala Leu Leu Thr Thr His Tyr Met Ala Glu

1425                1430                1435                1440

Ala Glu Ala Val Cys Asp Arg Val Ala Ile Met Val Ser Gly Arg Leu

                1445                1450                1455

Arg Cys Ile Gly Ser Ile Gln His Leu Lys Ser Lys Phe Gly Lys Asp

            1460                1465                1470

Tyr Leu Leu Glu Met Lys Val Lys Asn Leu Ala Gln Val Glu Pro Leu

        1475                1480                1485

His Ala Glu Ile Leu Arg Leu Phe Pro Gln Ala Ala Arg Gln Glu Arg

    1490                1495                1500

Tyr Ser Ser Leu Met Val Tyr Lys Leu Pro Val Glu Asp Val Gln Pro
1505                1510                1515                1520

Leu Ala Gln Ala Phe Phe Lys Leu Glu Lys Val Lys Gln Ser Phe Asp
                1525                1530                1535

Leu Glu Glu Tyr Ser Leu Ser Gln Ser Thr Leu Glu Gln Val Phe Leu
            1540                1545                1550

Glu Leu Ser Lys Glu Gln Glu Leu Gly Asp Phe Glu Glu Asp Phe Asp
        1555                1560                1565

Pro Ser Val Lys Trp Lys Leu Leu Pro Gln Glu Glu Pro
    1570                1575                1580

<210> 2
<211> 4743
<212> DNA
<213> Homo sapiens

<300>
<308> AB020629
<309> 1999-06-16

<300>

<303> DNA

<304> 5

<305> 6

<306> 355-364

<400> 2

atgaggaaga gaaagatcag tgtgtgtcaa caaacttggg ccttattatg caagaacttt 60

cttaaaaaat ggagaatgaa aagagagtcc ttaatggaat ggctgaattc attgctccta 120

ctactttgtt tgtatatata tcctcatagt catcaagtaa atgatttttc ttcactgctt 180

accatggacc tgggacgggt agatacattt aatgaatcca gattttctgt tgtatacaca 240

cctgtcacca acacgaccca acagataatg aataaagtag cctctactcc cttcctggca 300

ggtaaagagg tcttgggact gccagatgag gaaagtatta aagaattcac agcaaattat 360

cctgaagaaa tagtaagagt cacctttact aatacatact catatcattt gaagttcttg 420

ctaggacatg gaatgccagc aaagaaggag cacaaggacc atacagctca ttgttatgaa 480

acaaatgaag atgtttactg tgaagtttca gtattttgga aggaaggttt tgtggctctt 540

caagctgcca ttaatgctgc tattatagaa atcacaacaa atcactcagt gatggaggag 600

ctgatgtcag ttactggaaa aaatatgaag atgcattcct tcattggtca atcaggagtt 660

ataactgatt tgtacctttt ttcctgcatt atttcatttt cctcattcat ttactatgca 720

tctgttaatg tcacaagaga gaggaaaagg atgaaggcct tgatgacaat gatgggtctt 780

cgggattcag cgttctggct ctcctggggt ttgctctatg ctggtttcat cttcattatg 840

gccctttctt tggcacttgt tataagatct acccagttta tcattttgtc tggcttcatg 900

gtagtcttca gcctctttct cctgtatgga ttatctttgg tagctttggc tttcttaatg 960

agcatcttgg taaagaaatc tttcctcacc ggcctggtcg tgttcctcct cactgtcttt 1020

tgggggtgtc tggggttcac atcactgtac agacaccttc ctgcatcctt ggagtggatt 1080

ttaagcttgc ttagtccctt tgccttcatg cttggaatgg cccagctttt acacttggac 1140

tatgatttga attctaatgc atttcctcat ccatcggacg gctcaaatct cattgtagca 1200

acaaatttca tgttggcatt tgacacttgc ctctatctgg cattggcgat ttactttgaa 1260

aaaattttgc caaatgaata tggacatcga cgtccacctt tgtttttcct gaagtcctca 1320

ttttggtctc aaacacaaaa gactgatcac gtggcccttg aagatgaaat ggatgccgat 1380

ccttcatttc atgactcttt tgaacaagcg cctccagaat tccaagggaa agaagccatc 1440

agaatcagaa atgttacaaa agaatataaa ggaaagcctg ataaaataga agccttgaaa 1500

gatctggtat ttgacattta cgaaggccaa atcactgcaa tacttggtca cagtggagct 1560

ggaaagtcaa cactgctaaa cattcttagt gggttgtctg ttcccaccaa aggttcagtc 1620

accatctata acaataagct ttcagaaatg gctgacctag aaaatctcag caagctgacc 1680

ggagtttgtc cacaatccaa tgtgcaattt gacttcctca ctgtaagaga aaacctcaga 1740

ctctttgcta aaataaaagg gattctgcca caagaagtgg ataaagagat tttcctgttg 1800

gatgaaccaa ctgctggatt ggatcccttt tcaagacacc aagtatggaa ccttctgaaa 1860

gaacgcaaaa cagaccgcgt gatcctcttc agtacccagt tcatggatga ggccgacatc 1920

ctggcggaca ggaaagtatt tctctcccaa gggaagctaa agtgcgcggg ctcttctttg 1980

tttctaaaga agaaatgggg gattggatat cacttaagct tgcagttaaa tgaaatatgt 2040

gttgaggaaa acataacatc acttgttaaa cagcacatcc ctgatgccaa attatcagcc 2100

aaaagcgaag gaaaacttat ttatacatta cccttagaaa gaacaaataa atttccagaa 2160

ctttacaagg atcttgatag ctatcctgac ctaggaattg agaattatgg tgtttccatg 2220

acaactttga atgaagtatt cctgaagcta gaaggaaaat ctacaattaa tgaatcggac 2280

attgctattt tgggagaagt acaagcggaa aaagctgacg acactgaaag gcttgttgag 2340

atggaacaag tcctctcttc acttaacaag atgagaaaga caataggtgg tgtggctctc 2400

tggcgacagc aaatctgcgc aattgcaagg gttcgcttgt taaagttaaa gcatgaaaga 2460

aaagctcttt tagcactgct attaattcta atggctggat tttgccctct tcttgtggag 2520

tataccatgg tgaaaatata tcaaaacagt tacacctggg aactttctcc tcatttgtat 2580

ttccttgctc ctggacaaca accacatgac cctctcactc aactactgat catcaataaa 2640

acaggggcaa gcattgatga ctttatacag tctgtggagc accagaacat agctttagaa 2700

gtggatgcat ttggaactag aaatggcaca gatgacccat cttataatgg agccatcaca 2760

gtgtgttgta atgaaaagaa ttacagcttt tcgttagcat gcaatgccaa aagattgaat 2820

tgcttcccag ttcttatgga cattgttagt aatgggctac ttggaatggt taaaccatca 2880

gtacatatcc gaactgaaag aagtacattt ttggagaatg gacaggacaa tccaatcgga 2940

ttcctggcat atatcatgtt ctggctggtt ttaacatcga gttgcccacc ttacattgcc 3000

atgagcagca tcgatgatta taagaacaga gctcggtccc agctacggat ttccggactc 3060

tccccttctg cttactggtt tgggcaggcg ctggtggatg tttccctgta cttcttggtc 3120

ttcgttttta tatatttaat gagctacatt tcaaacttcg aagacatgct acttacaata 3180

attcatatta ttcaaatccc atgtgctgtt ggttattcct tttccctcat cttcatgaca 3240

tacgtgattt ccttcatctt tcgcaagggg agaaaaaata gtggcatttg gtcattttgt 3300

ttctatgttg tcactgtatt ctctgtggct ggatttgcgt tcagtatctt cgaaagtgat 3360

attccattta tcttcacttt tttaatacca cctgccacaa tgattggctg tttgttctta 3420

tcttctcatc ttctcttttc ttctctcttt tctgaagaac gaatggatgt acagccattt 3480

ctggtattcc taattccttt ccttcatttt atcatttttc tttttactct tcgatgtctg 3540

gaatggaagt ttggaaagaa atcaatgaga aaggatcctt tctttagaat ttctccaaga 3600

agtagtgatg tgtgtcaaaa tccagaagaa ccagaaggag aggatgaaga tgttcagatg 3660

gaaagagtga gaacagcaaa tgccttgaat tctactaatt ttgatgagaa gccagtcatc 3720

attgccagct gtctacgcaa ggagtatgca gggaagagga aaggctgttt ttccaagagg 3780

aagaataaga tagccacgag aaatgtctcc ttctgtgtta gaaaaggtga agttttagga 3840

ttattaggac acaatggagc tggtaaaagc acatccatta aggtgataac tggagacaca 3900

aaaccaactg ctggacaagt gctactgaaa gggagcggtg gaggggatgc cctggagttc 3960

ctggggtact gccctcagga gaacgcgctg tggcccaacc tgacagtgag gcagcacctg 4020

gaggtgtacg ccgccgtgaa agggctgagg aaaggggatg ctgaggttgc catcacacgg 4080

ttagtggatg cgctcaagct gcaggaccag ctgaagtctc cggtgaagac cttgtcagag 4140

ggaataaaga gaaagctgtg ctttgtcctg agcatactgg ggaacccgtc agtggtgctt 4200

ctggatgagc cgtcgaccgg gatggacccc gaggggcagc agcaaatgtg gcaggccatc 4260

cgggccacct ttagaaacac ggaaaggggt gccctcctaa ccacccacta catggcagag 4320

gctgaggccg tgtgtgaccg agtggccatc atggtatctg ggaggttgag atgtatcggt 4380

tccatccaac acctgaaaag caaatttggc aaagattacc tgctggagat gaaggtgaag 4440

aacctggcac aagtggagcc cctccatgca gagatcctga ggcttttccc ccaggctgct 4500

cggcaggaaa ggtactcctc tctgatggtt tataagttgc cagtggaaga tgtgcaacct 4560

ttagcccaag ctttcttcaa attagagaag gttaaacaga gctttgacct agaggagtac 4620

agcctctcac agtctaccct ggagcaggtt ttcctggagc tctccaagga gcaggagctg 4680

ggtgattttg aggaggattt tgatccctca gtgaagtgga agctcctccc ccaggaagag 4740

cct 4743

## Description

### Technical Field

The present invention relates to a novel use of a drug transporter. More specifically, it relates to a novel use of an ABC transporter known as ABCA8, and further relates to a method for screening a compound promoting or inhibiting the activity of ABCA8, and a use as a drug for overcoming drug resistance.

### Background Art

In order that a drug takes effects as a medicine, after the drug entered a body, it must exist at a target site in an appropriate beneficial concentration for a sufficient period of time. A drug metabolizing enzyme and a drug transporter control the kinetic profile of a drug (absorption, distribution, metabolism, excretion, and the actual concentration of a drug at a target site) in the body, and determine the overall pharmacological effect of the drug. When the effective concentration of a drug is low at a target site, the pharmacological effect of the drug cannot be expected. On the other hand, when the concentration of a drug is high at a target site, it is predicted that adverse reactions will occur.

Accordingly, in order to obtain a good pharmacological effect from a drug, the actual concentration of the drug at a target site should be controlled by the synergic action of a drug transporter (uptake and excretion) and a drug metabolizing enzyme.

A drug transporter is expressed in epithelial cells of the small intestine or the kidneys, hepatic cells, and cerebral vascular endothelial cells, and it plays an extremely important role in the kinetics of a drug in the body. Such a drug transporter is expressed in small intestine epithelial cells or cerebral vascular endothelial cells, and it has a great influence on the bioavailability of a drug that is orally or parenterally administered, or transportation of the drug to the central nervous system. In the meantime, in the field of cancer chemotherapy, it has been known that when P-glycoprotein (MDR1) or GS-X pump (MRP) is excessively expressed, cancer cells become resistant to anticancer agents. Such MDR1 and MRP are transporters belonging to an ATP-binding cassette (ABC) transporter family.

It has been clarified that the ABC transporter family has an ATP-binding cassette or a characteristic transmembrane domain, and that it is associated with the transportation of endogenous substances depending on ATP, foreign substances or metabolites thereof, which depends on ATP. Many ABC protein genes have been found in recent years, and 50 or more genes have been known at the present moment.

It is being clarified that the ABC transporter is associated with absorption of a drug by the small intestine or transportation of the drug to the brain.

As stated above, in order that a drug takes effect as a medicine, the role of a drug transporter is important.

It is an object of the present invention to provide a protein having a drug transport activity as described above, a method for screening a compound promoting or inhibiting the activity of a drug transporter, a compound obtained by the method, an antibody against a drug transporter, a pharmaceutical composition comprising the same, or the like.

### Disclosure of the Invention

As a result of intensive studies, the present inventors have analyzed a protein, which is expressed by a DNA having a certain nucleotide sequence obtained by screening EST databases (Genebank database) , and they have found that this protein has a drug transport activity, thereby completing the present invention.

That is to say, the present inventors have conducted BLAST search in Genebank, and they have screened for a protein having a transmembrane domain and ABC, thereby completing the present invention that will be described later.

Moreover, in order to clarify the function of a drug transporter known as ABCA8 (Drug Kinetics, Xenobio. Metanol. And Dispos. 15(1): 8-19 (2002); Hito ABC Transporter Idenshi no Shin Meimei Ho (A new method of naming a human ABC transporter gene)), the present inventors have progressively studied the function of the drug transporter, and they have found that the above drug transporter has an activity to transport organic anionic compounds such as conjugated bilirubin or indoxyl sulfate, thereby completing the present invention.

That is to say, the present invention provides a protein having a drug transport activity which comprised an amino acid sequence identical or substantially identical to the amino acid sequence represented by SEQ ID NO: 1, or a protein having a drug transport activity which comprises an amino acid sequence having deletions, substitutions or additions of one or more amino acids of the amino acid sequence represented by SEQ ID NO: 1, or a partial peptide, an amide, an ester or a salt thereof.

In addition, the present invention provides a protein having an organic anion transport activity which comprises an amino acid sequence identical or substantially identical to the amino acid sequence represented by SEQ ID NO: 1, or a protein having an organic anion transport activity which comprises an amino acid sequence having deletions, substitutions or additions of one or more amino acids of the amino acid sequence represented by SEQ ID NO: 1, or a partial peptide, an amide, an ester or a salt thereof.

Moreover, the present invention provides a method for screening a compound that promotes or inhibits the activity of a protein comprising an amino acid sequence identical or substantially identical to the amino acid sequence represented by SEQ ID NO: 1, or a partial peptide, an amide, an ester or a salt thereof, which is characterized by that the method uses a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

Furthermore, the present invention provides a method for screening a compound that is a substrate for a protein comprising an amino acid sequence identical or substantially identical to the amino acid sequence represented by SEQ ID NO: 1, or a partial peptide, an amide, an ester or a salt thereof, which is characterized by that the method uses a protein comprising an amino acid sequence identical or substantially identical to the amino acid sequence represented by SEQ ID NO: 1, or a protein having an amino acid sequence having deletions, substitutions or additions of one or more amino acids of the amino acid sequence represented by SEQ ID NO: 1, or a partial peptide, an amide, an ester or a salt thereof.

Still further, the present invention provides a kit for screening a compound that promotes or inhibits the activity of a protein comprising an amino acid sequence identical or substantially identical to the amino acid sequence represented by SEQ ID NO: 1, or a partial peptide, an amide, an ester or a salt thereof, which is characterized by that it uses a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

In addition, the present invention provides a kit for screening a compound that is a substrate for a protein comprising an amino acid sequence identical or substantially identical to the amino acid sequence.represented by SEQ ID NO: 1, or a partial peptide, an amide, an ester or a salt thereof, which is characterized by that it uses a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

Moreover, the present invention provides a compound that promotes or inhibits the activity of a protein comprising an amino acid sequence identical or substantially identical to the amino acid sequence represented by SEQ ID NO: 1, or a partial peptide, an amide, an ester or a salt thereof, or a compound that is a substrate for a protein comprising an amino acid sequence identical or substantially identical to the amino acid sequence represented by SEQ ID NO: 1, or a partial peptide, an amide, an ester or a salt thereof, which are obtained by the above described screening method, or using the above described screening kit.

Furthermore, the present invention provides a pharmaceutical composition, which comprises,
a protein having a drug transport activity which comprises an amino acid sequence identical or substantially identical to the amino acid sequence represented by SEQ ID NO: 1, or a protein having a drug transport activity comprising an amino acid sequence having deletions, substitutions or additions of one or more amino acids of the amino acid sequence represented by SEQ ID NO: 1, or a partial peptide, an amide, an ester or a salt thereof, or
a protein having an organic anion transport activity which comprises an amino acid sequence identical or substantially identical to the amino acid sequence represented by SEQ ID NO: 1, or a protein having an organic anion transport activity comprising an amino acid sequence having deletions, substitutions or additions of one or more amino acids of the amino acid sequence represented by SEQ ID NO: 1, or a partial peptide, an amide, an ester or a salt thereof.

Still further, the present invention provides a pharmaceutical composition comprising the above described compound or a salt thereof.

In addition, the present invention provides an antibody against a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

Moreover, the present invention provides a recombinant vector comprising a DNA encoding a protein comprising an amino acid sequence identical or substantially identical to the amino acid sequence represented by SEQ ID NO: 1, or a protein comprising an amino acid sequence having deletions, substitutions or additions of one or more amino acids with respect to the amino acid sequence represented by SEQ ID NO: 1.

Furthermore, the present invention provides a transformant transformed with the above described recombinant vector.

Still further, the present invention provides a method for producing a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, which is characterized by that the method comprises culturing the above described transformant, generating and accumulating a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, and collecting the above protein.

### Brief Description of the Drawings

Figure 1 shows the time course of uptake of ¹⁴C-estradiol-β-glucuronide into oocytes;
Figure 2 is the Michaelis-Menten equation of uptake of ¹⁴C-estradiol-β-glucuronide;
Figure 3 shows the results of a hydropathy analysis of the drug transporter of the present invention;
Figure 4 shows the influence of an ATP concentration on uptake of ¹⁴C-estradiol-β-glucuronide into oocytes;
Figure 5 shows the effects of various compounds to inhibit the uptake of ¹⁴C-estradiol-β-glucuronide into oocytes;
Figure 6 shows uptake of various compounds into oocytes; and
Figure 7 shows the time course of uptake of ³H-indoxyl sulfate into oocytes and the effect of probenecid to inhibit the uptake of ³H-indoxyl sulfate into oocytes.

### Best Mode for Carrying Out the Invention

The present invention will be described in detail below.

A protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ IDNO: 1 that is used in the present invention (hereinafter, in the present specification, referred to as the drug transporter of the present invention, at times) will be explained.

The above protein may be derived from, for example, humans or other hematherms, or may be a synthetic protein. It may also be what is called a recombinant protein obtained by the method for producing the drug transporter of the present invention, which will be described later.

In the present invention, a DNA obtained by screening EST-databases obtained from Kazusa DNA Research Institute is used, and a recombinant protein produced from the DNA is used. The DNA has been cloned as one of full-length cDNAs derived from human brain, and has been deposited with NCBI (National Center for Biotechnology Information, USA) under deposition No. AB020629. The DNA has 5677 base pairs. Moreover, the information on the DNA can be acquired from http://www.kazusa.or.jp/huge/gfpage/KIAA0822/.

In the present specification, the term "substantially identical" is used to mean that the activities of proteins, such as a drug transport activity (e.g., ABC transport activity), are substantially identical to each other. With a portion of amino acids deleted, substituted or added in a protein, the protein having such a deletion, substitution or addition is substantially identical to a protein that does not have such a deletion, substitution or addition.

In general, when a protein comprises an amino acid sequence having approximately 80% or more, and preferably 90% or more homology with the whole of amino acid sequence represented by SEQ ID NO: 1, it is understood that the protein is substantially identical to a protein comprising the amino acid sequence represented by SEQ ID NO: 1. Accordingly, a protein comprising an amino acid sequence having deletions, substitutions or additions of one or more amino acids (preferably 1 to 20, more preferably 1 to 10, andmost preferably several amino acids) of the amino acid sequence represented by SEQ ID NO: 1 is also substantially identical to the protein comprising the amino acid sequence represented by SEQ ID NO: 1.

A transporter having an organic anion transport activity is an example of the drug transporter of the present invention. Organic anion means an organic compound having an anionic group. Examples of such an organic anion may include an organic anion anticancer agent and an organic anion anti-HIV agent. Examples of such an organic anion anticancer agent may include methotrexate, irinotecan, monoglutathionylthiotepa, and arsenic trioxide. Examples of such an organic anion anti-HIV agent may include 9-(2-phosphonylmethoxyethyl) adenine (PMER), indinavir, nelfinavir, ritonavir, and saquinavir.

As a protein comprising an amino acid sequence identical or substantially identical to the amino acid sequence represented by SEQ ID NO: 1, or a protein comprising an amino acid sequence having deletions, substitutions or additions of one ormore amino acids of the amino acid sequence represented by SEQ ID NO: 1, there can be used those produced by using a transformant that is transformed with a DNA containing a DNA encoding a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1.

As a DNA encoding a protein comprising an amino acid sequence identical or a substantially identical to the amino acid sequence represented by SEQ ID NO: 1, or a protein comprising an amino acid sequence having deletions, substitutions or additions of one or more amino acids of the amino acid sequence represented by SEQ ID NO: 1, a DNA having the nucleotide sequence represented by SEQ ID NO: 2 can be used. In addition, there may also be used a DNA hybridizing with the DNA represented by SEQ ID NO: 2 under stringent conditions, and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1.

An example of the DNA hybridizing with the DNA represented by SEQ ID NO: 2 under stringent conditions may include a DNA comprising a nucleotide sequence having approximately 90% or more, pref erably approximately 95% or more, andmorepreferably approximately 98% ormore homologywith the nucleotide sequence represented by SEQ ID NO: 2. Hybridization can be carried out according to known methods or methods equivalent thereto, such as Molecular Cloning (Molecular Cloning, 2nd, J. Sambrook et al., Cold Spring Harbor Lab. Press, 1989). When a commercially available library is used, hybridization can be carried out according to the manual attached thereto. The term "stringent conditions" is used in the present specification to mean hybridization conditions such that when a probe is labeled with DIG DNA Labeling (manufactured by Roche Diagnostics) as an example, the DNA probe of the present invention is hybridized with the target DNA by Southern blot hybridization in which hybridization is carried out in a DIG Easy Hyb solution (manufactured by Roche Diagnostics) at 32°C and themembrane is then washed in a 0.1 × SSC solution containing 0.1% [x/v] SDS (1 × SSC: 0. 15 M NaCl and 0.015 M sodium citrate) at 40°C.

In order to clone a DNA comprising the nucleotide sequence represented by SEQ ID NO: 2, or a DNA hybridizing with the DNA represented by SEQ ID NO: 2 under stringent conditions and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1, the DNA may be amplified by PCR using synthetic DNA primers having a suitable nucleotide sequence corresponding to a portion of protein substantially identical to a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1. Otherwise, DNA incorporated into a suitable vector may be hybridized with a labeled DNA fragment or synthetic DNA encoding a part or the whole region of a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1, and then selection may be carried out. Hybridization can be carried out by the method described in Molecular Cloning 2nd (J. Sambrook et al. , Cold Spring Harbor Lab. Press, 1989) or the like. When a commercially available library is used, hybridization can be carried out according to the manual attached thereto. Transcription of the nucleotide sequence of DNA can be carried out by known methods such as the Gapped duplex method or the Kunkel method, or methods equivalent thereto, using a known kit such as a SuperScript II reverse transcriptase kit (Invitrogen). The DNA encoding a cloned polypeptide can be used directly, after digestion by restriction enzymes or addition of a linker thereto, as desired. The DNA may have a translation initiation codon ATG at a 5'-terminal side thereof, and a translation termination codon, TAA, TGA or TAG, at a 3'-terminal side thereof. These translation initiation codon and translation termination codon can be added using a suitable synthetic DNA adaptor.

A recombinant vector comprising DNA encoding a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1 can be produced by methods known in the present technical field. For example, the recombinant vector can be produced by (1) cutting out a DNA fragment containing the DNA represented by SEQ ID NO: 2, or the DNA hybridizing with the DNA represented by SEQ IDNO: 2 under stringent condition, and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence representedby SEQ IDNO : 1, and (2) ligating the DNA fragment to the downstream of a promoter in a suitable expression vector. Examples of a vector used herein may include plasmids derived from *Escherichia coli* (e.g., pBR322, pBR325, pUC18 or pUC118), plasmids derived from *Bacillus subtilis* (e.g., pUB110, pTP5 or pC194), plasmids derived from yeasts (e.g., pSH19 or pSH15), bacteriophages such as a λ phage, and animal viruses such as retrovirus, vaccinia virus or baculovirus. Any promoter can be used as a promoter used in the present invention, as long as it is a suitable promoter that is compatible with a host used in the expression of a gene. When the host is *Escherichia coli,* examples of a preferred promoter may include a trp promoter, a lac promoter, a recA promoter, a λP_{L} promoter, a lpp promoter, a T7 promoter, a T3 promoter, and an araBAD promoter. When the host is *Bacillus,* examples of a preferred promoter may include an SPO1 promoter, a penP promoter, an XYL promoter, an HWP promoter, and a CWP promoter. When the host is *Bacillus subtilis,* examples of a preferred promoter may include an SPO1 promoter, an SPO2 promoter, and a penP promoter. When the host is a yeast, examples of a preferred promoter may include a PHO5 promoter, a PGK promoter, a GAP promoter, and an ADH promoter. When an animal cell is used as a host, examples of a promoter may include an SRα promoter, an SV40 promoter, an LTR promoter, a CMV promoter, and an HSV-TK promoter. When an insect cell is used as a host, examples of a preferred promoter may include a polyhedrin promoter and an OplE2 promoter.

Other than the above items, an enhancer, a splicing signal, a poly A addition signal, a selective marker, an SV40 origin of replication (hereinafter abbreviated as SV40 ori, at times) or the like, which are known in the present technical field, may also be added to an expression vector, if desired. Moreover, if necessary, a fusion protein consisting of the protein encoded by the DNA of the present invention and other proteins (e.g., glutathione-S-transferase and protein A) can also be expressed. Such a fusion protein is cleaved with a site-specific protease, so that it can be divided into each protein.

Examples of a host cell used herein may include *Escherichia, Bacillus,* a yeast, an insect cell, an insect, and an animal cell. Specific examples of *Escherichia* may include *Escherichia coli* K12 DH1 (Proc. Natl. Acad. Sci. USA, Vol. 60, 160 (1968)), JM103 (Nucleic Acids Research, Vol. 9, 309 (1981)), JA221 (Journal of Molecular Biology, Vol. 120, 517 (1978)), HB101 (Journal of Molecular Biology, Vol. 41, 459 (1969)), DH5α, and JM109. Examples of *Bacillus* used herein may include *Bacillus subtilis* MI114 (Gene, Vol. 24, 255 (1983)), 207-21 [(Journal of Biochemistry, Vol. 95, 87 (1984)), and *Bacillus brevis.* Examples of a yeast used herein may include *Saccharomyces cerevisiae* AH22, AH22R-, NA87-11A, DKD-5D and 20B-12, *Schizosaccaromyces pombe* NCYC1913 and NCYC2036, *Pichia pastoris,* and *Hansenula polymorpha.* Examples of animal cells used herein may include monkey cells COS-7, Vero, Chinese hamster cells CHO (hereinafter abbreviated as CHO cells), dhfr gene-deficient Chinese hamster cells CHO (hereinafter abbreviated as CHO (dhfr-) cells), mouse L cells, mouse AtT-20 cells, mouse myeloma cells, rat GH3 cells, human FL cells and HEK293 cells.

The above described host cells can be transformed by methods known in the present technical field. For example, methods of transforming host cells are described in the following publications: Proc. Natl. Acad. Sci. USA, Vol. 69, 2110 (1972); Gene, Vol. 17, 107 (1982); Molecular & General Genetics, Vol. 168, 111 (1979); Methods in Enzymology, Vol. 194, 182-187 (1991); Proc. Natl. Acad. Sci. USA, Vol. 75, 1929 (1978); Saibo Kogaku Bessatsu 8 (Cell Technology, Suppl. 8) Shin Saibo Kogaku Jikken Protocol (New Cell Technology Experiment Protocol), 263-267 (1995) (Shujunsha Co., Ltd.); and Virology, Vol. 52, 456 (1973).

The above obtained transformant transformed with an expression vector containing a gene of a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, which comprises a DNA represented by SEQ ID NO: 2, or a DNA hybridizing with the DNA represented by SEQ ID NO: 2 under stringent condition, and encoding a protein substantially identical to a protein comprising an amino acid sequence identical or substantially identical to the amino acid sequence represented by SEQ ID NO: 1 or a protein substantially identical to a protein comprising an amino acid sequence having deletions, substitutions or additions of one or more amino acids of the amino acid sequence represented by SEQ ID NO: 1, is cultured by a known method. For example, when a host is *Escherichia,* the culture is generally carried out at approximately 15°C to 43°C for about 12 to 48 hours, and aeration or stirring may be added, if necessary. When a host is *Bacillus,* the culture is generally carried out at approximately 30°C to 40°C for about 12 to 100 hours, and aeration or stirring may be added, if necessary. When a transformant whose host is a yeast is cultured, the culture is generally carried out at approximately 20°C to 35°C for about 24 to 100 hours in a medium whose pH is adjusted about 5 to 8, and aeration or stirring may be added, if necessary. When a transformant whose host is an animal cell is cultured, the culture is generally carried out at approximately 30°C to 40°C for about 24 to 100 hours in a medium whose pH is adjusted about 6 to 8, and aeration or stirring may be added, if necessary.

In order to separate and purify a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, for example, there is applied a method of collecting cell mass or cells by a known method after the culture, suspending them in a suitable buffer, disintegrating the cell mass or cells by ultrasonic wave, lysozyme and/or freezing-thawing, and obtaining a crude extract of protein by centrifugal separation or filtration. Protein-denaturing agents such as urea or guanidine hydrochloride, or surfactants such as Triton X-100 (registered trademark) may also be added in the buffer. When the protein is secreted in the culture solution, cell mass or cells are separated from the supernatant by a known method after completion of the culture, and the supernatant is then collected. The thus obtained culture supernatant or the protein contained in the extract can be purified by a combined use of known separation and purification methods. The thus obtained polypeptide (protein) of the present invention can be converted into salts by known methods or methods equivalent thereto. In contrast, when the polypeptide (protein) of the present invention is obtained in the form of salts, the salts can be converted into free or other salts. Moreover, the protein generated by the transformant is reacted with a suitable protease such as trypsin or chymotrypsin before or after purification, so that it can be fragmentated, if necessary. Furthermore, the protein is reacted with a protein-modifying enzyme such as kinase, so that it can be modified, as necessary. The presence of the drug transporter or a salt thereof of the present invention can be determined by various binding assays, an enzyme immunoassay using a specific antibody, or the like.

The above described drug transporter of the present invention can be used to screen a compound as described later, or can be used to prepare an antibody. Moreover, by using a transformant transformed with a recombinant vector containing DNA encoding the drug transporter of the present invention, an extracorporeal circulatory artificial organ can be produced.

Furthermore, the drug transporter of the present invention is a protein capable of specifically transporting drugs, especially, organic anions such as an organic anion anticancer agent or organic anion anti-HIV agent. Accordingly, the drug transporter of the present invention can be used as a pharmaceutical composition. For example, the drug transporter of the present invention can be used as a pharmaceutical composition to administer an anticancer agent or immunosuppressive agent site-specifically or to eliminate poisonous substances. More specifically, a transmembrane domain region is cut out of the drug transporter of the present invention, so as to produce a protein consisting of a drug and an ATP-binding cassette region, or an antibody specific for a target site is led into the target site, so that a drug can be transported site-specifically. Moreover, a pharmaceutical composition comprising the drug transporter of the present invention and an organic anion anticancer agent or organic anion anti-HIV agent may be prepared and used.

Furthermore, since the drug transporter of the present invention has an activity to transport conjugated bilirubin, it can be used as a pharmaceutical composition for treating liver malfunction.

Still further, since the drug transporter of the present invention has an activity to transport indoxyl sulfate, it can be used as a pharmaceutical composition for treating kidney malfunction.

What is more, the drug transporter of the present invention can be used to screen a compound that can be a substrate for the drug transporter of the present invention. Transport efficiency by the drug transporter of the present invention is evaluated in advance on a compound that is being developed, so that the bioavailability of the compound can be assumed.

The method for screening a compound of the present invention will be described below.

The screening method of the present invention is a method for screening a compound that promotes or inhibits the activity of a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, using the protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, obtained as described above.

A protein used in the method for screening a compound of the present invention generally has a carboxyl group (-COOH) or a carboxylate (-COO-) at the C-terminus. However, the C-terminus may also be in the form of an amide (-CONH₂) or ester (-COOR). Examples of R in the ester may include C₁₋₆ alkyl groups such as methyl, ethyl, n-propyl, isopropyl or n-butyl, C₃₋₈ cycloalkyl groups such as cyclopentyl or cyclohexyl, C₆₋₁₂ aryl groups such as phenyl or α-naphthyl, and C₇₋₁₄ aralkyl groups including phenyl- C₁₋₂ alkyl groups such as benzyl or phenetyl, or α-naphthyl-C₁₋₂ alkyl groups such as α-naphthylmethyl, as well as pivaloyloxymethyl ester that is generally used as ester for oral administration.

In a case where the above protein has a carboxyl group (or carboxylate) other than at the C-terminus, proteins whose carboxyl group is amidated or esterified may also be used. In this case, as an ester, the above described C-terminal ester may be used, for example. Moreover, the scope of the protein of the present invention may also include proteins in which the amino group of methionine residue at the N-terminus is protected by a protecting group (e.g., a C₁₋₆ acyl group such as a formyl group or acetyl group), proteins in which the glutamic acid residue at the N-terminus generated by cleaving *in vivo* is pyroglutaminated, proteins in which OH, COOH, NH₂, SH or the like on the side chains of amino acids in a molecule are protected by an appropriate protecting group (e.g., a C₁₋₆ acyl group such as a formyl group or acetyl group), conjugated proteins such as what is called glycoproteins to which a sugar chain is bound, and the like.

The above partial peptide means a partial peptide of the above protein. Any partial peptide can be used, as long as it has a substantially homogenous activity to that of the protein. For example, there is used a peptide, which has an amino acid sequence consisting of at least 20 or more, preferably 50 or more, more preferably 70 or more, further more preferably 100 or more, and most preferably 200 or more amino acids of the constitutional amino acid sequence of a protein comprising an amino acid sequence identical to or substantially identical to the amino acid sequence represented by SEQ ID NO: 1, and has a biological activity substantially homologous to, for example, that of a protein comprising an amino acid sequence identical or substantially identical to the amino acid sequence represented by SEQ ID NO: 1. Moreover, the above partial peptide generally has a carboxyl group (-COOH) or a carboxylate (-COO-) at the C-terminus. However, as with the above described protein of the present invention, its C-terminal may be in the form of an amide (-CONH₂) or an ester (-COOR). Furthermore, as with the above described protein of the present invention, the scope of the above partial peptide may include peptides in which the amino group of methionine residue at the N-terminus is protected by a protecting group, peptides in which the glutamyl group generated by cleaving the N-terminus *in vivo* is pyroglutaminated, peptides in which a substituent on the side chain of amino acid in a molecule is protected by an appropriate protecting group, conjugated peptides such as what is called glycopeptides to which a sugar chain is bound, and the like.

As salts of the above protein or a partial peptide thereof, it is particularly preferable to be physiologically acceptable acid addition salts. Examples of such salts may include salts combined with inorganic acid (e.g., hydrochloric acid, phosphoric acid, hydrobromic acid, sulfuric acid) , and salts combined with organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The screening method of the present invention is characterized by that it uses a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

In order to screen a compound that promotes or inhibits the activity of the above protein, or a partial peptide, an amide, an ester or a salt thereof, a knownmethod can be applied. More specifically, the drug transport activity of the above protein, or a partial peptide, an amide, an ester or a salt thereof is determined. Thereafter, a compound that is a candidate of the compound that promotes or inhibits the activity of the above protein, or a partial peptide, an amide, an ester or a salt thereof is added thereto, and the drug transport activity of the mixture is determined. Thereafter, the activities of both cases are compared with each other.

A specific method comprises: injecting into cells DNA encoding the above protein or a partial peptide, cDNA to the DNA, or RNA obtained by treating the cDNA with reverse transcriptase; incubating the cells with a labeled compound (e.g., a compound labeled with a radioactive substance, enzyme, fluorescent substance, etc.); and after a certain period of time, determining a radioactivity, enzyme activity or fluorescence intensity that is taken into the cells. Moreover, screening of a compound that promotes or inhibits the above activity of the present invention is carried out by mixing a candidate compound into the above system, incubating the mixture, determining the radioactivity, enzyme activity or fluorescence intensity, and comparing it with the result in case that no candidate compounds are mixed.

Furthermore, a bead to which a transformant transformed by a recombinant vector containing DNA encoding the above protein or a partial peptide is placed in a microplate having multiple wells (e.g., 96 wells), and it is incubated with a labeled compound (e.g., a compound labeled with a radioactive substance, enzyme, fluorescent substance, etc.). After a certain period of time, the radioactivity, enzyme activity or fluorescence intensity that is taken into the transformed cells is determined. Screening of a compound that promotes or inhibits the above activity of the present invention is carried out by mixing a candidate compound into the above system, incubating the mixture, determining a radioactivity, enzyme activity or fluorescence intensity, and comparing it with the result in case that no candidate compounds are mixed.

As stated above, labeling of a compound can be carried out using a radioactive substance, enzyme, fluorescent substance or the like. Enzyme labeling or fluorescence labeling can be carried out without using radioisotope facility. As described above, the screening method of the present invention can be carried out using a compound labeled with a radioactive substance, enzyme or fluorescent substance. However, methods of labeling a compound are not limited to those described herein, the labeling can be carried out by any known method.

Various compounds are used as candidate compounds described above. Examples of a candidate compound may include a peptide, a protein, a non-peptide compound, a synthetic compound, and a compound contained in a fermented product, cell extract, plant extract or animal tissue extract, but the examples are not limited thereto. These compounds may be either novel compounds or known compounds.

The screening kit of the present invention comprises materials for carrying out the above screening method. For example, it comprises a protein or a partial peptide, an amide, an ester or a salt thereof for carrying out the screening method, and also comprises cells used in screening.

The compound that promotes or inhibits the activity of the drug transporter or a partial peptide, an amide, an ester or a salt thereof of the present invention, which is obtained by the screening method of the present invention or using the screening kit of the present invention, is selected from a group consisting of compounds subjected to the above test. The compound promotes or inhibits the drug transport activity of the above described drug transporter, or a partial peptide, an amide, an ester or a salt thereof of the present invention. Such a compound can be used to control the pharmacological effect of a drug. For example, where the pharmacological effect of a certain drug is intended to be improved, when the above described drug transporter, or a partial peptide, an amide, an ester or a salt thereof of the present invention has an effect to promote the uptake of the drug into cells, a compound that promotes the activity of the drug transporter is administered, and thereby the pharmacological effect of the drug is improved. On the other hand, where a drug has a narrow range between a level taking a pharmacological effect and a level causing adverse reactions, a compound that inhibits the activity of the above protein, or a partial peptide, amide, ester or salt thereof is administered, so that the adverse reactions can be reduced.

Moreover, where the above described drug transporter, or a partial peptide, an amide, an ester or a salt thereof of the present invention has an effect to promote excretion of a certain drug from cells, an inhibitor is administered to improve the pharmacological effect of the drug.

The pharmaceutical composition of the present invention comprises a compound that promotes or inhibits the activity of the above described drug transporter or a partial peptide, an amide, an ester or a salt thereof of the present invention, or a salt thereof. Salts of the above compound are the same as those of the above described protein.

The pharmaceutical composition of the present invention is prepared by common means, and it can be administered orally or parenterally. It can be administered, for example, as a tablet, a capsule, an elixir, a microcapsule, an aseptic solution, a suspension, or the like. Since the thus obtained preparation is safe and low-toxic, it can be administered to humans or hematherms (e.g., a mouse, a rat, a rabbit, a sheep, a swine, a bovine, a horse, a fowl, a cat, a dog, a monkey, a chimpanzee, etc.). The dosage of the compound or a salt thereof depends on purposes.

An antibody against a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, will be explained below.

An antibody against the protein of the present invention comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof may be either a polyclonal antibody or monoclonal antibody, as long as it can recognize a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof. The antibody can be produced by a known method of producing an antibody or antiserum, using, as an antigen, a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

Methods of producing a monoclonal antibody and a polyclonal antibody will be explained below.

### [Production of monoclonal antibody]

### (a) Production of a monoclonal antibody-generating cell

The protein of the present invention is, for example, subcutaneously injected to a site of a hematherm capable of generating an antibody, by itself or together with a carrier oradiluent. For immunization, in order to enhance the ability to generate an antibody, Freund' s complete adjuvant or Freund's incomplete adjuvant may also be administered. The protein is administered generally once every 2 to 6 weeks, about 2 to 10 times in total. Examples of a hematherm used herein may include a monkey, a rabbit, a dog, a guinea pig, a mouse, a rat, a sheep, a goat, and a chicken. A mouse and a rat are preferably used. To produce monoclonal antibody-generating cells, an individual, of which antibody levels are elevated, is selected from hematherms, such as mice, immunized with an antigen, and the spleen or the lymph node is collected therefrom 2 to 5 days after the final immunization. Antibody-generating cells contained in those organs are fused with the myeloma cells of the same or different species of animal, so as to prepare monoclonal antibody-generating hybridomas. The above fusion procedure can be carried out by a known method such as the Kohler-Milstein method [Nature, 256, 495 (1975)]. Examples of a fusion promoter may include polyethylene glycol (PEG) and Sendai virus, and PEG is preferably used.

Examples of a myeloma cell may include those of hematherms, such as NS-1, P3U1, SP2/0 or AP-1. Of these, P3U1 is preferably used. A preferred ratio of the number of the used antibody-generating cells (spleen cells) to the number of myeloma cells is between 1 : 1 and 20 : 1. PEG (preferably PEG 1000 to PEG 6000) is added thereto in a concentration of approximately 10% to 80%, and incubation is carried out at 20°C to 40°C, and preferably at 30°C to 37°C for 1 to 10 minutes, so that cell fusion can be efficiently carried out. Various methods can be applied to screen a monoclonal antibody-generating hybridoma. Examples of such a screening method may include: a method comprising adding a hybridoma culture supernatant to a solid phase (e.g., a microplate) to which a protein antigen is adsorbed directly or together with a carrier, and then adding thereto an anti-immunoglobulin antibody (where a fusion cell is derived from a mouse, an anti-mouse immunoglobulin antibody is used) or protein A that is labeled with a radioactive substance, enzyme, or the like so as to detect a monoclonal antibody binding to the solid phase; and a method comprising adding a hybridoma culture supernatant to a solid phase to which an anti-immunoglobulin antibody or protein A is adsorbed, and then adding thereto a protein antigen labeledwith a radioactive substance, enzyme, or the like, so as to detect a monoclonal antibody binding to the solid phase. Hybridomas can be selected by known methods or methods equivalent thereto. Selection of hybridomas can be carried out generally in a medium for animal cells, to which HAT (hypoxanthine, aminopterin, thymidine) is added. Any medium may be used as a growth medium, as long as a hybridoma can grow therein. For example, an RPMI 1640 medium containing 1% to 20%, preferably 10% to 20% fetal bovine serum, GIT medium containing 1% to 10% fetal bovine serum (Wako Pure Chemical Industries, Ltd.), or serum free medium for hybridoma culture (SFM-101, Nissui Pharmaceutical Co., Ltd.) can be used. The culture temperature is generally 20°C to 40°C, and preferably approximately 37°C. The culture time is generally 5 days to 3 weeks, and preferably 1 to 2 weeks. The culture can generally be carried out in 5% carbon dioxide gas. The antibody titer of a hybridoma culture supernatant can be determined by reacting a labeled protein with the culture supernatant, and then measuring the activity of a labeling agent binding to the antibody.

### (b) Purification of monoclonal antibody

Separation and purification of a monoclonal antibody can be carried out by known methods, such as methods of separating and purifying an immunoglobulin [ e.g., salting-out, alcohol precipitation, isoelectric precipitation, electrophoresis, adsorption-desorption method using an ion exchanger (e.g., DEAE), ultracentrifugation, gel filtration, a specific purification method of collecting only an antibody with an antigen-bound solid phase, or active adsorbent such as protein A or protein G, and then dissociating the binding, so as to obtain an antibody].

### [Production of polyclonal antibody]

The polyclonal antibody of the present invention can be produced by known methods or methods equivalent thereto. For example, a hematherm is immunized with an immunizing antigen (protein antigen) itself or a complex with a carrier protein in the same manner as in the above method of producing a monoclonal antibody. Thereafter, the blood is collected from the hematherm, followed by separation and purification of an antibody. With regard to a complex of an immunizing antigen and a carrier protein used for immunization of a hematherm, any type of carrier protein can be used and any mixing ratio of the carrier can be applied, as long as an antibody can be efficiently produced against the hapten immunized in the form of cross-linkage with the carrier. For example, there is used a method of coupling bovine serum albumin, globulin, hemocyanin or the like with a hapten at a weight ratio of approximately 0.1 to 20 : 1, and preferably approximately 1 to 5 : 1. In order to couple a hapten with a carrier, various condensing agents can be used. An active ester reagent containing glutaraldehyde, carbodiimide, maleimide active ester, a thiol group or a dithiopyridyl group may be used as such a condensing agent. In addition, a DNA is produced by fusing a DNA expressing a fusion protein with a DNA expressing the drug transporter of the present invention, and a recombinant vector is then produced using the fused DNA. The recombinant vector is incorporated into a suitable host to transform it, so that a fusion protein is produced. The thus obtained fusion protein may be used as an antigen. The fusion protein is administered to a site of a hematherm capable of generating an antibody, by itself or together with a carrier or a diluent. For immunization, in order to enhance the ability to generate an antibody, Freund' s complete adjuvant or Freund' s incomplete adjuvant may also be administered. The protein is administered generally once every 2 to 6 weeks, about 3 to 10 times in total. A polyclonal antibody can be collected fromthe blood of the hematherm immunized by the above described method. The polyclonal antibody titer of the antiserum can be determined by reacting a labeled protein with the antiserum, and then measuring the activity of the labeling agent binding to the antibody. Separation and purification of the polyclonal antibody can be carried out by the same method of separating and purifying an immunoglobulin as described in the above separation of purification of a monoclonal antibody.

The above produced antibody can be used in the same manner as for the above described compound that inhibits the activity of the above protein, a partial peptide, an amide, an ester or a salt thereof. In this case, if the antibody is a monoclonal antibody, it has an advantage in that it is easily used because the above protein, a partial peptide, an amide, an ester or a salt thereof has an individual inhibition activity.

Moreover, the above produced antibody can be used in various diagnoses regarding drug transporters. For example, a human-derived sample (e.g., biopsy) is collected, and the above antibody can be used to examine what kind of transporter and what amount of transporter is expressed therein.

The extracorporeal circulatory artificial organ of the present invention will be explained below.

The extracorporeal circulatory artificial organ of the present invention is characterized by that it comprises hollow fibers to which cells are attached, and that the cells are the above described transformants.

The liver is an organ conducting metabolism and regulation in the metabolism. It has enormous complicated functions necessary for a living body, such as metabolism of various compounds, detoxication of endogenous or exogenous substances, or excretion. In the case of the conventional artificial organs, for example, activated carbons are placed in a device for dialysis or the like, and a drug is adsorbed thereto. However, such an artificial organ has a problem that it eliminates even substances necessary for a living body, such as erythrocytes or thrombocytes, as well as a drug of interest.

A transformant transformed with a recombinant vector containing a DNA encoding the above protein is attached to the extracorporeal circulatory artificial organ of the present invention. Accordingly, the present extracorporeal circulatory artificial organ enables drug-specific elimination, so that it can be used to treat drug poisoning.

For example, since the drug transporter of the present invention has an activity to transport conjugated bilirubin, a transformant transformed with a recombinant vector containing a DNA encoding the drug transporter of the present invention can be used as an artificial liver for treatment of liver malfunction. This is to say, the artificial liver can be applied to patients with livermalfunction. For example, human liver-derived HepG2 cells are co-transfected with the cDNAof the present invention and cDNAof P450 that specifically decomposes a drug, so that the drug is eliminated and decomposed, and that the drug poisoning can be treated.

Moreover, since the drug transporter of the present invention has an activity to transport indoxyl sulfate, a transformant transformed with a recombinant vector containing the DNA encoding the drug transporter of the present invention can be used as an artificial kidney for treatment of kidney malfunction. This is to say, the artificial kidney can be applied to patients with kidney malfunction.

Furthermore, as a hollow fiber used in the artificial organ of the present invention, a porous hollow fiber that has conventionally been used as an artificial kidney, artificial liver or the like is used. Examples of material for such a hollow fiber may include hydrophilic polyolefin and cuprammonium rayon. The membrane thickness of a hollow fiber is generally between 5 and 70 µm, and preferably between 10 and 50 µm. The internal diameter of the membrane is generally between 100 and 400 µm, and preferably between 180 and 330 µm. Furthermore, the pore size is generally between 0.01 and 3 µm, and preferably between 0.1 and 0.3 µm. The membrane area of a hollow fiber used for the artificial organ of the present invention is generally between 150 and 300 cm², and preferably between 160 and 200 cm².

The present invention will be further specifically described in the following examples. However, needless to say, the scope of the present invention is not limited by the following examples.

### Example 1

A mature female *Xenopus* purchased from Hamamatsu Animal was bred under normal conditions. Thereafter, stage Voocytes were selected therefrom, and the cells were incubated in a modified Barth' s solution (MBS) containing 88 mM NaCl, 1 mM KC1, 2.4 mM NaHCO₃, 15 mM Tris-C1, 0.3 mM CaNO₃, 0.82 mM MgSO₄, 0.41 mM CaCl₂, 10 µg/ml penicillin and 10 µg/ml streptomycin. Thereafter, cRNA was prepared by a known method using a DNA having the nucleotide sequence represented by SEQ ID NO: 1, and an aqueous solution containing the obtained cRNA was injected into the mature *Xenopus* oocytes by microinjection. For such microinjection, cRNA whose 5'-terminus was capped or 50 nl of water was injected into the oocytes *in vitro,* using an automatic injector (IM200J, Narishige, Japan). The thus injected oocytes were cultured at 17°C for 3 days in MBS, while exchanging the medium with a new one every day. 3 days after the culture, the oocytes expressed a drug transporter.

It should be noted that the above describedmicroinj ection method is disclosed in Ohki G, Miyoshi T, Murata M, Ishibashi K, Imai M, Suzuki M. A calcium-activated cation current by an alternatively spliced form of Trp3 in the heart. J Biol Chem. 2000 Dec 15; 275 (50): 39055-60.

The used cRNA was obtained by reverse transcription of cDNA from Kazusa DNA Research Institute, which has been cloned as one of full-length cDNAs obtained from human brain and has been deposited with NCBI (National Center for Biotechnology Information, USA) under accession No. AB020629.

The cultured oocytes (n = 10 to 20) were mixed with 2 to 3 ml of a medium and an isotope-labeled compound, and the mixture was then placed in a small incubator. Small amounts of the cells were serially sampled. Each oocyte was washed with a fresh MBS 3 times, and it was then dissolved in a 10% SDS solution at room temperature for 30 minutes. As such an isotope-labeled compound, ¹⁴C-estradiol-β-glucuronide that is a model probe for MRP (multidrug resistance associated protein), a member of an ABC transporter family, was used. Subsequently, a scintillation cocktail (ACS2, manufactured by Amersham) was added thereto, and then, a radioactivity taken into the oocyte was measured with a liquid scintillation counter (LSC-3500, manufactured by Aloka).

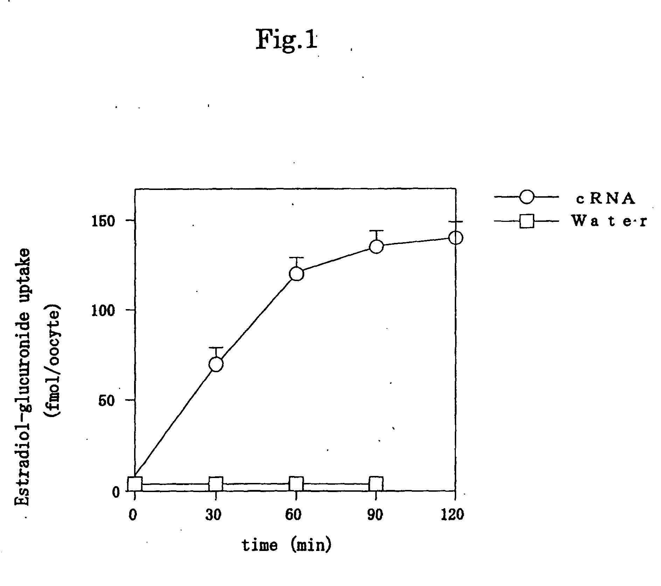

Figure 1 shows results obtained when ¹⁴C-estradiol-β-glucuronide was used. In this case, the concentration of ¹⁴C-estradiol-β-glucuronide in the medium was 37 nM. As shown in Figure 1, until about 60 minutes have elapsed, the uptake of ¹⁴C-estradiol-β-glucuronide in the oocytes to which cRNA was injected was linearly increased. The intake of ¹⁴C-estradiol-β-glucuronide into the cells was 138 ± 5.5 fmol/hr/egg in about 60 minutes have elapsed. In a group of oocytes to which water was injected, the uptake of ¹⁴C-estradiol-β-glucuronide was almost negligible (4 fmol/hr/egg) . From the results shown in Figure 1, it is found that the drug transporter of the present invention has an activity to take the above compound into the cells. Since it is difficult to label bilirubin by a radioisotope, an experiment was carried out using estradiol. From the fact that the drug transporter of the present invention has an activity to take ¹⁴C-estradiol-β-glucuronide into the cells, it is clear that it has an activity to take conjugated bilirubin into the cells. Moreover, the fact that ¹⁴C-estradiol-β-glucuronide has transporting properties that are similar to those of conjugated bilirubin is described in Suzuki Y, Sugiyama Y. Excretion of GSSG and glutathione conjugates mediated by MRP 1 and cMOAT/MRP2. Semin Liver Dis 1998; 18 (4): 359-376.

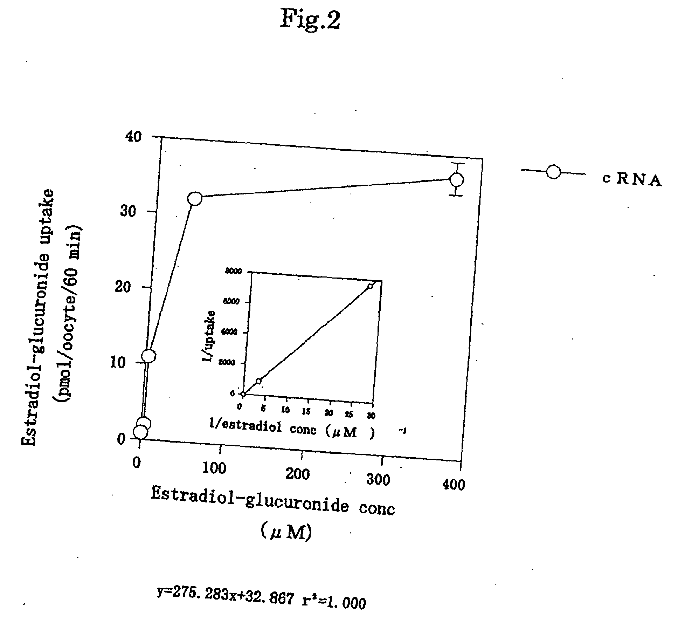

The uptake of ¹⁴C-estradiol-β-glucuronide is shown in kinetics and the following Michaelis-Menten equation (refer to Figure 2). Km and Vmax were 30.4 µM and 8.37 pmol/hr/egg, respectively.

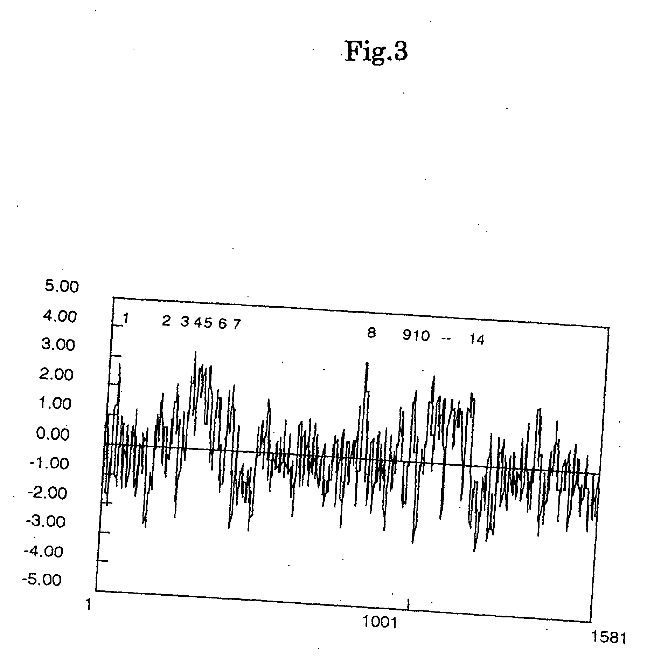

The expressed protein was subjected to a hydropathy analysis. The results of the analysis are shown in Figure 3. As is clear from the results shown in Figure 3, the drug transporter of the present invention has 14 transmembrane domains and 2 ATP-binding cassettes.

### Example 2

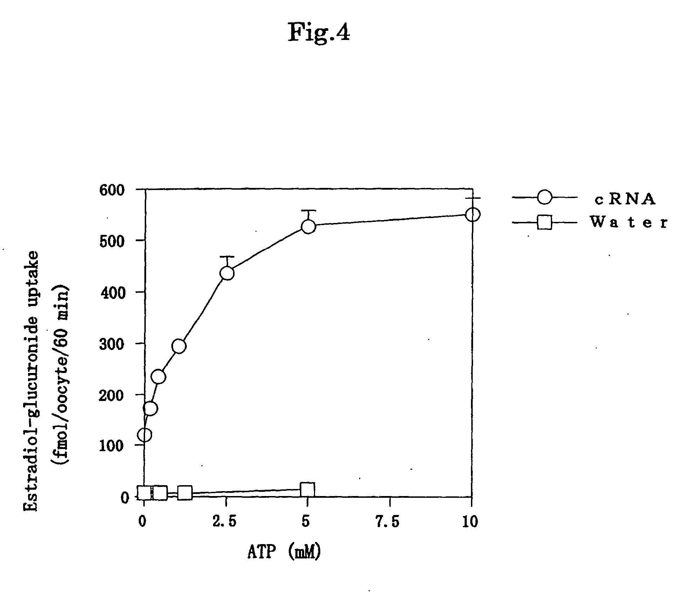

A protein encoded by a DNA comprising the nucleotide sequence represented by SEQ IDNO: 2 has anATP-binding cassette. Thus, a test was made to see ATP dependency of the uptake of ¹⁴C-estradiol-β-glucuronide. In the test, ATP was added in the above determination of the uptake of ¹⁴C-estradiol-β-glucuronide, and the same operation was then carried out to determine the uptake of ¹⁴C-estradiol-β-glucuronide. The results are shown in Figure 4.

As shown in Figure 4, as the concentration of ATP was increased, the uptake of ¹⁴C-estradiol-β-glucuronide into the cells was also increased. The uptake of the compound was increased with the increase of ATP concentration, and it was increased until the ATP concentration reached 5 mM. These results match the data of other genes belonging to the ABC transporter family.

### Example 3

An inhibition test was carried out on the protein encoded by a DNA comprising the nucleotide sequence represented by SEQ ID NO: 2. In the test, in the above described determination of the uptake of ¹⁴C-estradiol-β-glucuronide, various types of compounds were previously added to a reaction medium, so as to examine whether or not the uptake of
¹⁴C-estradiol-β-glucuronide was inhibited by the compounds. The used compounds are the following:
Verapamil: a substrate for MDR (an additive amount: 1 mM)
Digoxin: a substrate for MDR (an additive amount: 0.25 mM)
MS-209: an inhibitor for MRP (an additive amount: 10 µM)
Probenecid: a substrate for OAT (an additive amount: 1 mM)
Cimetidine: a substrate for OCT (an additive amount: 1 mM)
MK-571: an inhibitor for MRP (an additive amount: 10 µM)
Ochratoxin A: a substrate for MRP (an additive amount: 50 µM)

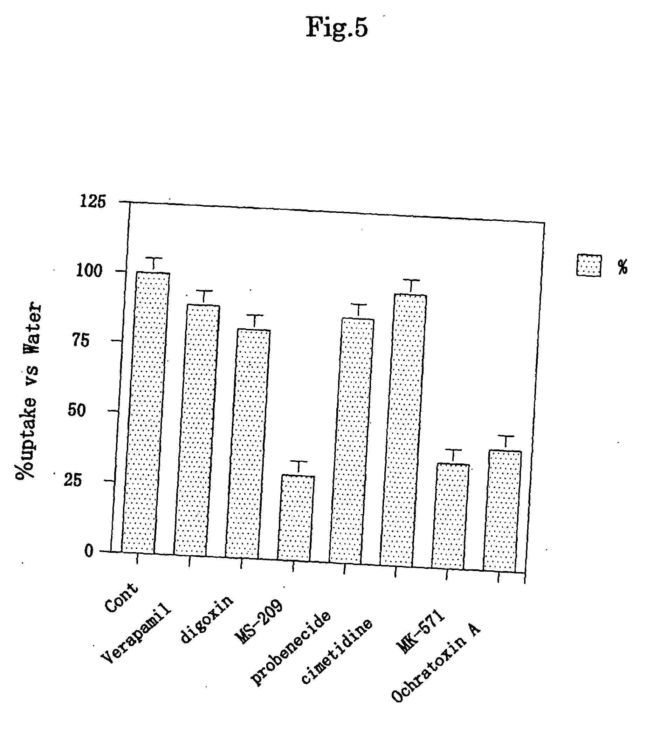

The results are shown in Figure 5. As shown in Figure 5, a cis-inhibitory effect was shown by addition of MK-571 or MS-209, and the uptake was decreased to approximately 30% by addition of these compounds. In addition, ochratoxin A also showed such an inhibitory effect. Moreover, verapamil and digoxin showed a small extent of inhibitory effect. Furthermore, probenecid also showed a small extent of inhibitory effect,but cimetidineshowed no inhibitory effect.

As is clear from these results, from the above described system , it is possible to make a system for detecting a compound that inhibits the activity of the drug transporter of the present invention. Using a DNA encoding the drug transporter of the present invention, it is possible to make a system for screening a compound that inhibits (or promotes) the activity of the drug transporter of the present invention.

Moreover, from the fact that MS-209 and MK-571 that have been developed as drugs for overcoming resistance of anticancer agents and ochratoxin A that is a substrate for MRP having an anion-transporting activity show inhibitory effects, it is shown that the protein encoded by a DNA comprising the nucleotide sequence represented by SEQ ID NO: 2 has an organic anion transport activity. Furthermore, it is suggested that the above protein can be used as a drug for overcoming the drug resistance of organic anion agents.

### Example 4

Subsequently, the uptake of various isotope-labeled compounds into cells was measured. Measurement was carried out in the same manner as in Example 1 with the exception that the following compounds were used.
LTC4 (leukotriene C4) : a substrate for MRP (an additive amount: 20 µM)
Taurocholic acid: a substrate forOAT (an additive amount: 100 µM)
TEA (tetraethylammonium): a substrate for OCT (an additive amount: 10 nM)
Digoxin: a substrate for MDR (an additive amount: 100 nM)
Doxorubicin: a substrate for MDR (an additive amount: 100 µg/ml)
PAH (para-aminohippuric acid): a substrate for MRP and OAT (an additive amount: 1 mM)

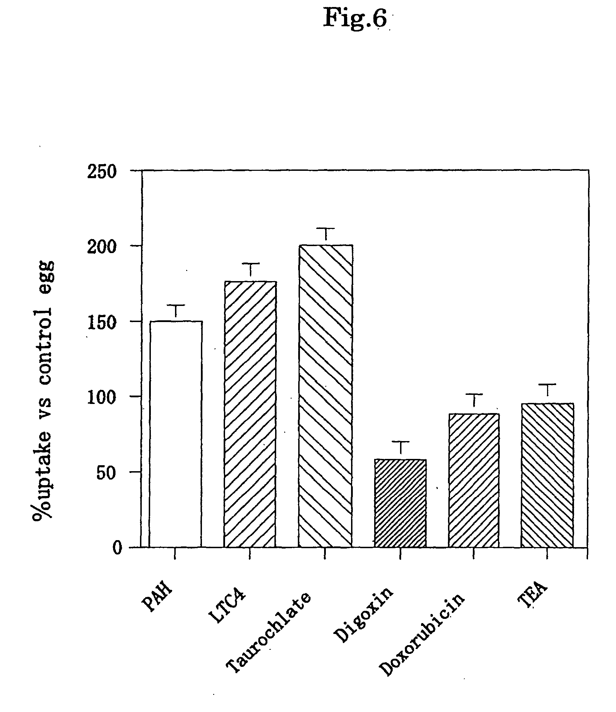

The results are shown in Figure 6. As shown in Figure 6, it was found that PAH, LTC-4 and taurocholic acid were taken into oocytes, but that digoxin was excreted therefrom (or its original uptake was inhibited).

Moreover, as is apparent from the results shown in Figure 6, it is found that the drug transporter of the present invention has a function to transport PAH, LTC4 and taurocholic acid that are substrates for MRP or OAT having an anion-transporting activity, but that it does not have a function to transport doxorubicin and TEA that are substrates for MDR or OCT having a cation-transporting activity.

### Example 5

Tissue distribution of the drug transporter of the present invention was studied by determining the tissue distribution by the Northern blotting method. This is to say, the tissue distribution was determined by the following method. MTN blot (manufactured by Clontech) was used as a transfer filter having human total RNA derived from various organs. The transfer filter was hybridized by random priming at a temperature of 68°C for 3 hours in a hybridization solution (Quickhyb, manufactured by Stratagene) containing a probe (the full length cDNA) labeled with [α-³²P] -dCTP. Subsequently, the filterwas washed with 0.1 × SSC and 0.5% SDS at a temperature of 55°C for 30 minutes. Thereafter, the filter was sandwiched between two sheet of sensitized paper (manufactured by Kbdac), and it was then exposed to an X-ray film at a temperature of -70°C. The results obtained by the Northern blotting method showed that the drug transporter of the present invention was detected in many tissues, and that it was detected in a large amount, especially in the heart and the small intestine (not shown in figures).

### Example 6

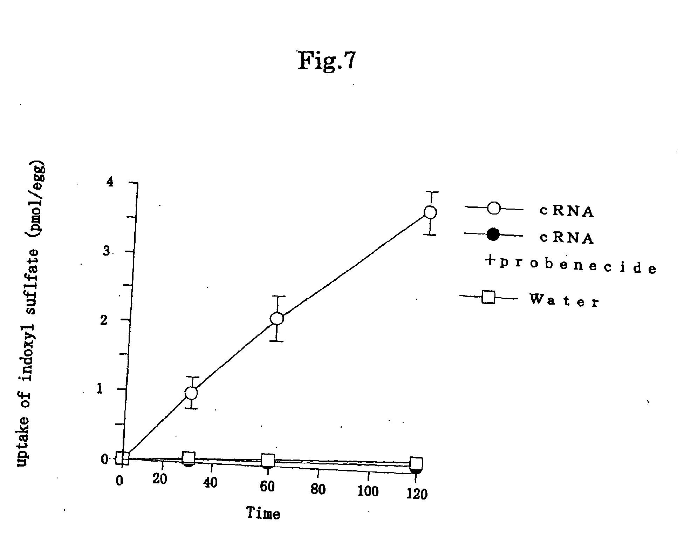

The same procedure was carried out as in Example 1 with the exception that ³H-labeled indoxyl sulfate, one of the urinary toxins, was isotope-labeled with ³H was used as a compound in a concentration of 2 µM. At the same time, a system to which probenecid was added in a concentration of 1 mM, and a system to which water was added instead of cRNA, were also subjected to the same test. The results are shown in Figure 7.

As shown in Figure 7, in the system to which water was inj ected, the uptake of ³H-indoxyl sulfate was hardly observed. In contrast, in the system to which cRNA was injected, the uptake of ³H-indoxyl sulfate was linearly increased with time. Moreover, by addition of probenecid, the uptake of ³H-indoxyl sulfate into the cells was decreased. The estimated Km was 25.5 µM, and Vmax was 5.5 pmol/hr/egg.

### Example 7

An antibody against the drug transporter of the present invention was produced. For an immunogen, a fusion protein obtained by fusing GST (glutathione-S-transferase) with a peptide corresponding to a portion from glutamic acid at position 1380 to glutamic acid at position 1579 of the amino acid sequence represented by SEQ ID NO: 1 was used as an antigen. Arabbit (Japanese white rabbit) at an age of 6 months or younger was used as an immune animal.

For immunization, an antigen solution obtained by dissolving 0.2 mg to 0.4 mg of antigen in a physiological saline solution was intracutaneously administered to the back of the rabbit 4 to 7 times every 2 weeks. Only for the first immunization, Freund's complete adjuvant (FCA) was used, but then, Freund's incomplete adjuvant (FIA) was used for all the subsequent immunizations. 10 days after the final immunization, antiserum was collected, and it was subjected to salting-out with a saturated ammonium sulfate solution. Subsequently, it was dialyzed with a phosphate buffered physiological saline (pH 7.4), so as to salt out an antibody.

Subsequently, a fused protein obtained by fusing a maltose binding protein (MBP) and a peptide corresponding to a portion from glutamic acid at position 1380 to glutamic acid at position 1579 of the amino acid sequence represented by SEQ ID NO: 1 was purified. The fused protein solution was substituted with a 0.5 M sodium bicarbonate-buffered saline (pH 8.3), and thereafter, it was injected into an NHS-activated sepharose (manufactured by Amersham) resin column so as to fix it to the resin. Unreacted active groups on the resin were inactivated with a 0.5 M monoethanolamine solution (pH 8.3). Subsequently, the salted out antibody solution was poured into the column, and it was washed with a phosphate buffered physiological saline (pH 7.4) in an amount of 6 times of the amount of the resin. Thereafter, a 0.1 M glycine hydrochloride solution (pH 2.7) was poured therein in an amount of 5 times of the amount of the resin. A solution eluted from the column was separated and immediately neutralized with a 1 M Tris solution (pH 9.0). Thereafter, it was dialyzed with a phosphate buffered physiological saline (pH 7.4), so as to obtain a specific antibody.

In all the above steps, the titer and specificity of the antibody were confirmed by the ELISA method.

As stated in detail above, the DNA encoding the drug transporter of the present invention can screen a compound that inhibits (or promotes) the activity of the drug transporter of the present invention. Moreover, the drug transporter of the present invention is useful as a reagent for screening a compound that inhibits or promotes the activity of the drug transporter of the present invention. Furthermore, it is also useful as a drug for overcoming the drug resistance of organic anion agents.

Still further, the drug transporter of the present invention has an ability to transport indoxyl sulfate that is one of the urinary toxin. If epithelial cells in which the drug transporter of the present invention is expressed could be obtained, the cells are cultured in hollow fibers, and there is a possibility that urinary toxins, which are hardly eliminated by an ordinary hemodialysis (having a high protein-binding fraction) , may be eliminated using the hollow fibers.

## Claims

1. A protein having a drug transport activity and comprising an amino acid sequence identical or substantially identical to the amino acid sequence represented by SEQ ID NO: 1, or a protein having a drug transport activity and comprising an amino acid sequence having deletions, substitutions or additions of one or more amino acids of the amino acid sequence represented by SEQ ID NO: 1, or a partial peptide, an amide, an ester or a salt thereof.

2. A protein having an organic anion transport activity and comprising an amino acid sequence identical or substantially identical to the amino acid sequence represented by SEQ ID NO: 1, or a protein having an organic anion transport activity and comprising an amino acid sequence having deletions, substitutions or additions of one or more amino acids of the amino acid sequence represented by SEQ ID NO: 1, or a partial peptide, an amide, an ester or a salt thereof.

3. The protein according to claim 2, or a partial peptide, an amide, an ester or a salt thereof, wherein said organic anion is an organic anion anticancer agent or an organic anion anti-HIV agent.

4. A DNA containing DNA comprising a nucleotide sequence encoding the protein according to any one of claims 1 to 3 or a partial peptide thereof.

5. The DNA according to claim 4 comprising the nucleotide sequence represented by SEQ ID NO: 2.

6. A recombinant vector containing a DNA encoding a protein comprising an amino acid sequence identical or substantially identical to the amino acid sequence represented by SEQ ID NO: 1, or protein comprising an amino acid sequence having deletions, substitutions or additions of one or more amino acids of the amino acid sequence represented by SEQ ID NO: 1.

7. The recombinant vector according to claim 6, wherein said DNA is
a DNA comprising the nucleotide sequence represented by SEQ ID NO: 2, or
a DNA hybridizing with the DNA represented by SEQ ID NO: 2 under stringent conditions, and encoding a protein comprising an amino acid sequence identical or substantially identical to the amino acid sequence represented by SEQ ID NO: 1, or a protein substantially identical to a protein comprising an amino acid sequence having deletions, substitutions or addition of one or more amino acids of the amino acid sequence represented by SEQ ID NO: 1.

8. A transformant transformed with the recombinant vector according to claim 6 or 7.

9. A method for producing a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof,
**characterized by** comprising the steps of:
culturing the transformant according to claim 8,
generating and accumulating a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, and
collecting said protein.

10. Amethod for screening a compound that promotes or inhibits the activity of a protein comprising an amino acid sequence identical or substantially identical to the amino acid sequence represented by SEQ ID NO: 1, or a partial peptide, an amide, an ester or a salt thereof,
**characterized by** using a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

11. A method for screening a compound that is a substrate for a protein comprising an amino acid sequence identical or substantially identical to the amino acid sequence represented by SEQ ID NO: 1, or a partial peptide, an amide, an ester or a salt thereof,
**characterized by** using a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

12. The screening method according to claim 10 or 11, wherein the protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, is produced by using a transformant transformed with a DNA containing DNA encoding a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1.

13. The screening method according to claim 12, wherein said DNA is
a DNA comprising the nucleotide sequence represented by SEQ ID NO: 2, or
a DNA hybridizing with the DNA represented by SEQ ID NO: 2 under stringent conditions, and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1.

14. A kit for screening a compound that promotes or inhibits the activity of a protein comprising an amino acid sequence identical or substantially identical to the amino acid sequence represented by SEQ ID NO: 1, or a partial peptide, amide, ester or salt thereof,
**characterized by** using a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

15. A kit for screening a compound that is a substrate for a protein comprising an amino acid sequence identical or substantially identical to the amino acid sequence represented by SEQ ID NO: 1, or a partial peptide, an amide, an ester or a salt thereof,
**characterized by** using a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

16. The screening kit according to claim 14 or 15, wherein the protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, is produced by using a transformant transformed with a DNA containing DNA encoding a protein comprising an amino acid sequence identical or substantially identical to the amino acid sequence represented by SEQ ID NO: 1, or protein having an amino acid sequence having deletions, substitutions or additions of one or more amino acids of the amino acid sequence represented by SEQ ID NO: 1.

17. The screening kit according to claim 16, wherein said DNA is
a DNA comprising the nucleotide sequence represented by SEQ ID NO: 2, or
a DNA hybridizing with the DNA represented by SEQ ID NO: 2 under stringent conditions, and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1.

18. A compound that promotes or inhibits the activity of a protein comprising an amino acid sequence identical or substantially identical to the amino acid sequence represented by SEQ ID NO: 1, or a partial peptide, an amide, an ester or a salt thereof, or
a compound that is a substrate for a protein comprising an amino acid sequence identical or substantially identical to the amino acid sequence represented by SEQ ID NO: 1, or a partial peptide, an amide, an ester or a salt thereof,
which is obtained by the screening method according to any one of claims 10 to 13, or using the screening kit according to any one of claims 14 to 17.

19. A pharmaceutical composition containing the compound according to claim 18 or a salt thereof.

20. A pharmaceutical composition, comprising,
a protein having a drug transport activity and comprising an amino acid sequence identical or substantially identical to the amino acid sequence represented by SEQ ID NO: 1, or a protein having a drug transport activity and comprising an amino acid sequence having deletions, substitutions or additions of one or more amino acids of the amino acid sequence represented by SEQ ID NO: 1, or a partial peptide, an amide, an ester or a salt thereof, or
a protein having an organic anion transport and comprising an amino acid sequence identical or substantially identical to the amino acid sequence represented by SEQ ID NO: 1, or a protein having an organic anion transport activity and comprising an amino acid sequence having deletions, substitutions or additions of one or more amino acids of the amino acid sequence represented by SEQ ID NO: 1, or a partial peptide, an amide, an ester or a salt thereof.

21. The pharmaceutical composition according to claim 20, further comprising an organic anion anticancer agent or an organic anion anti-HIV agent.

22. An antibody against a protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof.

23. The antibody according to claim 22, wherein the protein comprising an amino acid sequence identical or substantially identical to the amino acid sequence represented by SEQ ID NO: 1, or a protein comprising an amino acid sequence having deletions, substitutions or additions of one or more amino acids of the amino acid sequence represented by SEQ ID NO: 1, or a partial peptide, an amide, an ester or a salt thereof, is produced by using a transformant transformed with a DNA containing DNA encoding a protein comprising an amino acid sequence identical or substantially identical to the amino acid sequence represented by SEQ ID NO: 1, or a protein compirising an amino acid sequence having deletions, substitutions or additions of one or more amino acids of the amino acid sequence represented by SEQ ID NO: 1.

24. The antibody according to claim 23, wherein said DNA is
a DNA comprising the nucleotide sequence represented by SEQ ID NO: 2, or
a DNA hybridizing with the DNA represented by SEQ ID NO: 2 under stringent conditions, and encoding a protein comprising an amino acid sequence identical or substantially identical to the amino acid sequence represented by SEQ ID NO: 1, or a protein substantially identical to a protein comprising an amino acid sequence having deletions, substitutions or additions of one or more amino acids of the amino acid sequence represented by SEQ ID NO: 1.

25. An extracorporeal circulatory artificial organ comprising hollow fibers to which cells are attached, **characterized by** that said cells are the transformed cells according to claim 8.

26. The extracorporeal circulatory artificial organ according to claim 25, which is applied to patients with kidney malfunction.

27. The extracorporeal circulatory artificial organ according to claim 25, which is applied to patients with liver malfunction.
